(19) Europäisches Patentamt
European Patent Office
Office européen des brevets

(11) **EP 3 915 476 B1**

(12) **EUROPEAN PATENT SPECIFICATION**

(45) Date of publication and mention
of the grant of the patent:
**26.02.2025 Bulletin 2025/09**

(21) Application number: **20770434.7**

(22) Date of filing: **05.03.2020**

(51) International Patent Classification (IPC):
***A61B 5/1455*** *(2006.01)*  ***A61B 5/00*** *(2006.01)*
***A61B 5/1477*** *(2006.01)*

(52) Cooperative Patent Classification (CPC):
**A61B 5/7214; A61B 5/14551; A61B 5/681**

(86) International application number:
**PCT/CN2020/077946**

(87) International publication number:
**WO 2020/182047 (17.09.2020 Gazette 2020/38)**

(54) **BLOOD OXYGEN DETECTION METHOD AND DEVICE**

BLUTSAUERSTOFFDETEKTIONSVERFAHREN UND -VORRICHTUNG

PROCÉDÉ ET DISPOSITIF DE DÉTECTION D'OXYGÈNE SANGUIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2019 CN 201910186108**

(43) Date of publication of application:
**01.12.2021 Bulletin 2021/48**

(73) Proprietor: **Huawei Technologies Co., Ltd.
Shenzhen, Guangdong 518129 (CN)**

(72) Inventors:
• **LI, Yue**
  **Shenzhen, Guangdong 518129 (CN)**
• **LI, Yan**
  **Shenzhen, Guangdong 518129 (CN)**
• **YANG, Bin**
  **Shenzhen, Guangdong 518129 (CN)**
• **LI, Jing**
  **Shenzhen, Guangdong 518129 (CN)**
• **ZHOU, Linfeng**
  **Shenzhen, Guangdong 518129 (CN)**

(74) Representative: **Gill Jennings & Every LLP
The Broadgate Tower
20 Primrose Street
London EC2A 2ES (GB)**

(56) References cited:
WO-A1-2016/015009    WO-A1-2017/133883
CN-A- 101 940 475     CN-A- 103 169 478
CN-A- 103 169 478     CN-A- 105 249 939
CN-A- 108 420 440     CN-A- 108 937 957
CN-A- 108 937 957     US-A1- 2016 367 154
US-B2- 7 469 157

## Description

## TECHNICAL FIELD

This application relates to the field of blood oxygen detection, and in particular, to a wrist pulse blood oxygen detection method and apparatus based on multi-channel and independent component analysis.

## BACKGROUND

Blood oxygen saturation (oxygen saturation, SpO2) is a concentration of blood oxygen in blood. The blood oxygen saturation is an important physiological parameter of respiratory circulation and describes an ability of the blood to carry and deliver oxygen. A metabolism process of a human body is a biological oxidation process, and oxygen required in the metabolism process enters the blood of the human body through a respiratory system. The oxygen entering the blood of the human body and deoxyhemoglobin (hemoglobin, Hb) in red blood cells are combined to form oxyhemoglobin HbO2, and then the oxyhemoglobin is delivered to cells of various parts of the human body. SpO2 is a percentage of HbO2 in the blood to a total hemoglobin capacity. In other words, SpO2 = HbO2/(HbO2 + Hb) x 100%. Usually, the percentage is about 98%.

Currently, in some pulse blood oxygen detection, because blood vessels are sparsely distributed on a wrist, and on a surface layer, many veins are distributed and a pulse blood oxygen signal is relatively weak, interference caused by the veins cannot be ignored in data collected by using a single red light channel and an infrared light channel. Consequently, a signal-to-noise ratio of the data is relatively low. In addition, it is sensitive to a relative motion between skin and a sensor when one detection point is used for measurement. Therefore, this easily leads to interference caused by the motion.

CN 103169478 A describes a blood oxygen measurement device which comprises a light emitting device, a light detecting device and a signal processing circuit, wherein the light emitting device is arranged on one side of an object to be detected and is used for emitting first wavelength light and second wavelength light, the light detecting device is arranged on the other side of the detected object relative to the light emitting device and comprises a first narrow-band light detector and a second narrow-band light detector, the first narrow-band light detector is used for receiving transmission light of the first wavelength light transmitting through the detected object and converting the transmission light into an electric signal corresponding to the first wavelength light, the second narrow-band light detector is used for receiving transmission light of the second wavelength light transmitting through the detected object and converting the transmission light into an electric signal corresponding to the second wavelength light, and the signal processing circuit is respectively coupled to an output end of the first narrow-band detector and an output end of a second narrow-band light detector and calculates oxyhemoglobin saturation. The blood oxygen measurement device effectively suppresses environment light, and meanwhile provides possibilities for improving capacities of the blood oxygen measurement device in terms of suppression of movement interference of a measured object.

CN 108937957 A describes a detecting method, device and equipment. The detecting equipment comprises light source equipment, an optical detector and an acceleration sensor. The method comprises the steps that pulse oximeter signals obtained after a plurality of pieces of detection light emitted by the optical detector on the light source equipment pass through the detected tissue are acquired; heart rate detection waves carrying interference signals are subjected to filtering processing with acceleration signals as the standard; a dynamic heart rate value and a reference signal are obtained according to the heart rate detection waves; blood oxygen detection waves are subjected to effective section extraction according to the reference signal; the blood oxygen detection waves are calculated through a preset algorithm, and a ratio value R is obtained; the blood oxygen saturability is obtained according to the ratio value R. According to the detecting scheme, the interference signals introduced due to motion are filtered out to avoid the influence of motion on the detection result. The heat rate detection waves are adopted as the reference signal to carry out effective section extraction on the blood oxygen detection waves, and the defect that due to the fact that the blood oxygen detection waves are weak in interference resisting capacity, bad influence is caused to the detection result is avoided.

WO 2016/015009 A1 describes a pulse oximeter device including a first light emitting element that emits red light, a second light emitting element that emits green light or IR light; and a sensor element that detects red and green (or IR) light and that outputs signals representing detected red and green (or IR) light. The pulse oximeter device further includes a flexible substrate, wherein the first light emitting element, the second light emitting element and the sensor element are formed on the flexible substrate. The sensor element is configured to detect the emitted red and green light transmitted through tissue containing blood, and in certain aspects, the sensor element is configured to detect the emitted red and green (or IR) light reflected by tissue containing blood. A signal processing element (e.g., a processor) receives and processes the signals representing detected red and green (or IR) light output by the sensor element to produce signals representing blood oxygenation content.

US 7469157 B2 describes a signal processor which acquires a first signal, including a first primary signal portion and a first secondary signal portion, and a second signal, including a second primary signal portion and a second secondary signal portion, wherein the first and second primary signal portions are correlated. The signals may be acquired by propagating energy through a medium and measuring an attenuated signal after transmission or reflection. Alternatively, the signals may be acquired by measuring energy generated by the medium. A processor generates a primary or secondary reference signal which is a combination, respectively, of only the primary or secondary signal portions. The secondary reference signal is then used to remove the secondary portion of each of the first and second measured signals via a correlation canceler, such as an adaptive noise canceler, preferably of the joint process estimator type. The primary reference signal is used to remove the primary portion of each of the first and second measured signals via a correlation canceler. The processor may be employed in conjunction with a correlation canceler in physiological monitors wherein the known properties of energy attenuation through a medium are used to determine physiological characteristics of the medium. Many physiological conditions, such as the pulse, or blood pressure of a patient or the concentration of a constituent in a medium, can be determined from the primary or secondary portions of the signal after other signal portion is removed.

US 2016/367154 A1 describes obtaining cardiovascular parameters using arterioles related transient time. In case the signal from larger blood vessels is measured geometrically relatively far from such a signal from smaller, arteriole-like blood vessels, said measured time difference may be evaluated by several ways in order to separate a PWTT component of time differences, obtained from said measured signals, from transient time component, including information about changes of diameter in arteriole-like vessels. It also may be effective defining more correctly the role of blood viscosity in monitored vascular condition of patient.

## SUMMARY

Embodiments of this application provide a blood oxygen detection method and apparatus. Alternating current/direct current decomposition is performed on red light signals and infrared signals collected at a plurality of collection points, an alternating current part is separated into a plurality of independent components by using an independent component analysis algorithm, and then correlation is performed between each independent component and a green light signal. In this way, interference of venous blood flows and capillaries can be effectively eliminated, a disadvantage of a low signal-to-noise ratio is made up, and an accuracy of a blood oxygen measurement on a wrist is increased.

According to a first aspect, a blood oxygen detection method is provided, as set out in claim 1.

In a possible implementation, the determining red light direct current data and a component signal of a red light alternating current signal based on the at least two red light signals; and determining infrared direct current data and a component signal of an infrared alternating current signal based on the at least two infrared signals includes: determining at least two red light direct current signals and at least two red light alternating current signals based on the at least two red light signals; determining the red light direct current data based on the at least two red light direct current signals; determining at least two infrared direct current signals and at least two infrared alternating current signals based on the at least two infrared signals; determining the infrared direct current data based on the at least two infrared direct current signals; and determining the component signal of the red light alternating current signal based on the at least two red light alternating current signals, and determining the component signal of the infrared alternating current signal based on the at least two infrared alternating current signals.

In a possible implementation, the determining the component signal of the red light alternating current signal based on the at least two red light alternating current signals, and determining the component signal of the infrared alternating current signal based on the at least two infrared alternating current signals includes: separating at least one component signal of the at least two red light alternating current signals by using an independent component analysis algorithm or a principal component analysis algorithm, to obtain at least one component signal of the red light alternating current signal, where the at least one component signal of the red light alternating current signal includes an arterial signal; and separating at least one component signal of the at least two infrared alternating current signals by using the independent component analysis algorithm or the principal component analysis algorithm, to obtain at least one component signal of the infrared alternating current signal, where the at least one component signal of the infrared alternating current signal includes an arterial signal.

In a possible implementation, the determining red light alternating current data based on the component signal of the red light alternating current signal and the green light signal; and determining infrared alternating current data based on the component signal of the infrared alternating current signal and the green light signal includes: performing filtering processing on the green light signal; and determining the red light alternating current data based on the component signal of the red light alternating current signal and a green light signal obtained after the filtering processing, and determining the infrared alternating current data based on the component signal of the infrared alternating current signal and the green light signal obtained after the filtering processing.

In a possible implementation, the determining red light alternating current data based on the component signal of the red light alternating current signal and the green light signal; and determining infrared alternating current data based on the component signal of the infrared alternating current signal and the green light signal includes: performing comparison based on the at least one component signal of the red light alternating current signal and the green light signal, and determining that data corresponding to at least one component signal, of the red light alternating current signal, closest to the green light signal is the red light alternating current data; and performing comparison based on the at least one component signal of the infrared alternating current signal and the green light signal, and determining that data corresponding to at least one component signal, of the infrared alternating current signal, closest to the green light signal is the infrared alternating current data.

In a possible implementation, the red light direct current data is an average value, a maximum value, a minimum value, or a median of the at least two red light direct current signals; and the infrared direct current data is an average value, a maximum value, a minimum value, or a median of the at least two infrared direct current signals.

In a possible implementation, the determining blood oxygen saturation based on the red light direct current data, the red light alternating current data, the infrared direct current data, and the infrared alternating current data includes: determining pulse blood oxygen based on the red light direct current data, the red light alternating current data, the infrared direct current data, and the infrared alternating current data; and querying a pre-configured comparison table based on the pulse blood oxygen, to determine the blood oxygen saturation.

According to a second aspect, a blood oxygen detection apparatus is provided as set out in claim 8.

In a possible implementation, the processor is further configured to: determine at least two red light direct current signals and at least two red light alternating current signals based on the at least two red light signals; determine the red light direct current data based on the at least two red light direct current signals; determine at least two infrared direct current signals and at least two infrared alternating current signals based on the at least two infrared signals; determine the infrared direct current data based on the at least two infrared direct current signals; determine the component signal of the red light alternating current signal based on the at least two red light alternating current signals; and determine the component signal of the infrared alternating current signal based on the at least two infrared alternating current signals.

In a possible implementation, the processor is further configured to: separate at least one component signal of the at least two red light alternating current signals by using an independent component analysis algorithm or a principal component analysis algorithm, to obtain at least one component signal of the red light alternating current signal, where the at least one component signal of the red light alternating current signal includes an arterial signal; and separate at least one component signal of the at least two infrared alternating current signals by using the independent component analysis algorithm or the principal component analysis algorithm, to obtain at least one component signal of the infrared alternating current signal, where the at least one component signal of the infrared alternating current signal includes an arterial signal.

In a possible implementation, the processor is further configured to: perform filtering processing on the green light signal; determine the red light alternating current data based on the component signal of the red light alternating current signal and a green light signal obtained after the filtering processing; and determine the infrared alternating current data based on the component signal of the infrared alternating current signal and the green light signal obtained after the filtering processing.

In a possible implementation, the processor is further configured to: perform comparison based on the at least one component signal of the red light alternating current signal and the green light signal, and determine that data corresponding to at least one component signal, of the red light alternating current signal, closest to the green light signal is the red light alternating current data; and perform comparison based on the at least one component signal of the infrared alternating current signal and the green light signal, and determine that data corresponding to at least one component signal, of the infrared alternating current signal, closest to the green light signal is the infrared alternating current data.

In a possible implementation, the red light direct current data is an average value, a maximum value, a minimum value, or a median of the at least two red light direct current signals. The infrared direct current data is an average value, a maximum value, a minimum value, or a median of the at least two infrared direct current signals.

In a possible implementation, the processor is further configured to: determine pulse blood oxygen based on the red light direct current data, the red light alternating current data, the infrared direct current data, and the infrared alternating current data; and query a pre-configured comparison table based on the pulse blood oxygen, to determine the blood oxygen saturation.

This application discloses a blood oxygen detection method and apparatus. More information is collected by using a plurality of measurement points, collected alternating current information is separated by using an independent component algorithm, and denoising is performed by using a green light signal, so that interference caused by venous blood flows, capillaries, and the like can be eliminated, a disadvantage of a low signal-to-noise ratio is made up, and accuracy of blood oxygen measurement on a wrist is increased.

## BRIEF DESCRIPTION OF DRAWINGS

FIG. 1a is a schematic diagram of a pulse blood oxygen meter according to the conventional technology;

FIG. 1b is a schematic diagram of another pulse blood oxygen meter according to the conventional technology;

FIG. 2 is a flowchart of a blood oxygen detection method according to an embodiment of this application;

FIG. 3 is a flowchart of another blood oxygen detection method according to an embodiment of this application;

FIG. 4 is a schematic diagram of a blood oxygen detection apparatus according to an embodiment of this application; and

FIG. 5 is a schematic diagram of another blood oxygen detection apparatus according to an embodiment of this application.

## DESCRIPTION OF EMBODIMENTS

The following describes the technical solutions in the embodiments of this application with reference to the accompanying drawings in the embodiments of this application.

This application is applied to wrist blood oxygen detection. Hemoglobin has different absorption rates for red light and infrared light. HbO2 absorbs more near-infrared light (infrared radiation, IR), and a wavelength of the IR is usually about 900 nm. Hb absorbs more red light, and a wavelength of the red light is usually about 600 nm. The red light and the infrared light are radiated into human tissue at the same time. Because blood flows of veins and other body tissue are relatively constant, absorption of light can be approximately considered as a fixed value. Arteries periodically expand with a pulse, and therefore a total blood volume per unit volume changes periodically. Therefore, absorption of the red light and infrared light by the arteries changes periodically with the pulse.

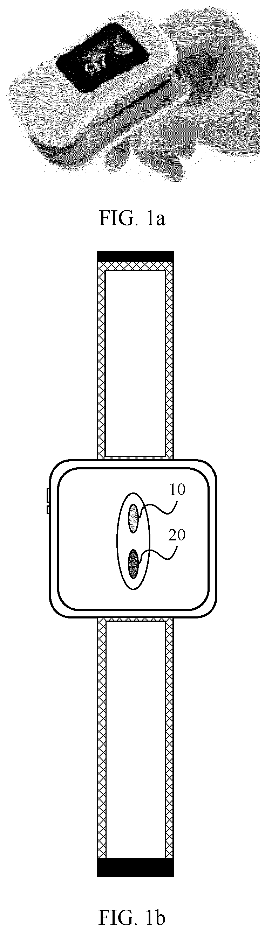

Some existing blood oxygen detection apparatuses use a finger clip type, and a finger of a measured person is put into a pulse oximeter of a finger clip type for measurement. As shown in FIG. 1a, in this solution, because the finger of the measured person needs to be clipped by the oximeter, the oximeter seriously interferes with a movement of the finger. In addition, it is more likely to cause discomfort when the finger is clipped for a long time. There are also some blood oxygen detection apparatuses using wrist-type single-channel measurement. A single-channel means that a signal is collected by using a single detection point. As shown in FIG. 1b, 10 is a red light sensor, and 20 is an infrared sensor. Pulse blood oxygen is detected by wearing the oximeter on the wrist. However, because blood vessels are sparsely distributed on the wrist and many veins are distributed on a surface layer, a pulse blood oxygen signal is relatively weak. Interference caused by the veins cannot be ignored in data collected by a single red light and infrared sensor. Consequently, a signal-to-noise ratio of the data is relatively low. In addition, it is sensitive to a relative motion between skin and a sensor when a single detection point is used for measurement. Therefore, this easily leads to interference caused by the motion.

To resolve the problem in the foregoing technology, in a detection method in this application, information about a plurality of detected parts is collected, and collected red light and infrared signals are divided into a red light alternating current signal, an infrared alternating current signal, a red light direct current signal, and an infrared direct current signal. Because signals collected by the sensor are a large quantity of original signals, data that can best reflect a current blood oxygen status needs to be extracted from the collected original signals. Therefore, in this application, red light alternating current data and infrared alternating current data are determined from the red light alternating current signal and the infrared alternating current signal through a correlation analysis with a green light signal. In addition, red light direct current data and infrared direct current data are determined by using the red light direct current signal and the infrared direct current signal. The red light direct current data in this application may be an average value, a maximum value, a minimum value, a median, or the like of a plurality of red light direct current signals. The infrared direct current data in this application may be an average value, a maximum value, a minimum value, a median, or the like of a plurality of infrared direct current signals. A green light signal is used to perform denoising on the red light alternating current data and the infrared alternating current data, so that interference caused by venous blood flows, capillaries, and the like can be eliminated. Finally, red light alternating current data, infrared alternating current data, red light direct current data, and infrared direct current data that are obtained after the denoising are used to calculate blood oxygen saturation. A disadvantage of a low signal-to-noise ratio is made up and accuracy of a blood oxygen measurement on the wrist is increased.

The following describes the technical solutions in the embodiments of this application with reference to the accompanying drawings in the embodiments of this application.

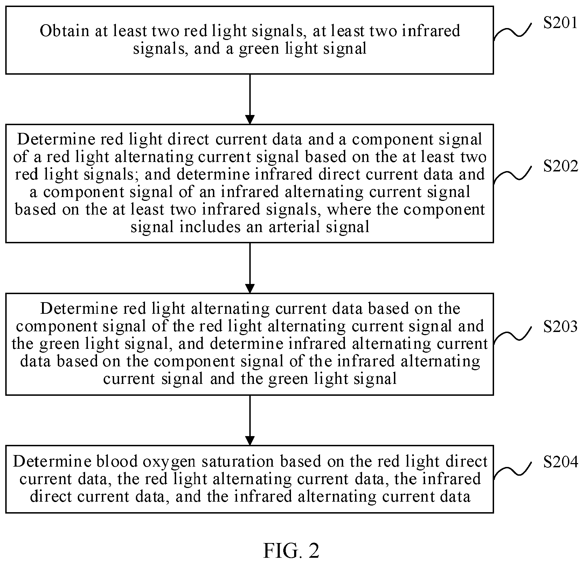

FIG. 2 is a flowchart of a blood oxygen detection method according to an embodiment of this application. As shown in FIG. 2, the method includes the following steps.

S201: Obtain at least two red light signals, at least two infrared signals, and a green light signal.

A signal of a measured user is collected by using at least two red light sensors, at least two infrared sensors, and a green light sensor. In an example, one detection point may include one red light sensor and one infrared sensor. In an embodiment, two detection points may be included, and each detection point obtains one red light signal and one infrared signal. In addition, a green light detection point is further included, and is configured to obtain a green light signal.

S202: Determine red light direct current data and a component signal of a red light alternating current signal based on the at least two red light signals; and determine infrared direct current data and a component signal of an infrared alternating current signal based on the at least two infrared signals, where the component signal includes an arterial signal.

Alternating current/direct current separation is performed on the at least two collected red light signals, to obtain the red light direct current data and the component signal of the red light alternating current signal. In addition, alternating current/direct current decomposition is performed on the at least two collected infrared signals, to obtain the infrared direct current data and the component signal of the infrared alternating current signal. In an example, the component signal includes the arterial signal. In another example, the component signal may alternatively include a capillary signal, another noise signal, or the like.

S203: Determine red light alternating current data based on the component signal of the red light alternating current signal and the green light signal, and determine infrared alternating current data based on the component signal of the infrared alternating current signal and the green light signal.

Compared with red light, green light can be absorbed by oxyhemoglobin and deoxyhemoglobin, a signal obtained by using the green light as a light source is better, and a signal-to-noise ratio is also better than that of another light source. Therefore, in this application, the green light is used for measurement, and is used as reference data of a pulse signal. In an example, there are a plurality of component signals, including a pulse signal. The green light signal is used to select the pulse signal from the plurality of component signals as alternating current data, so as to determine the red light alternating current data and the infrared alternating current data.

S204: Determine blood oxygen saturation based on the red light direct current data, the red light alternating current data, the infrared direct current data, and the infrared alternating current data.

In this application, more information is collected by using a plurality of measurement points, then collected alternating current information is separated by using an independent component analysis algorithm, and denoising is performed by using the green light signal, so that interference caused by venous blood flows, capillaries, and the like can be eliminated, a disadvantage of a low signal-to-noise ratio is made up, and accuracy of a blood oxygen measurement on a wrist is increased.

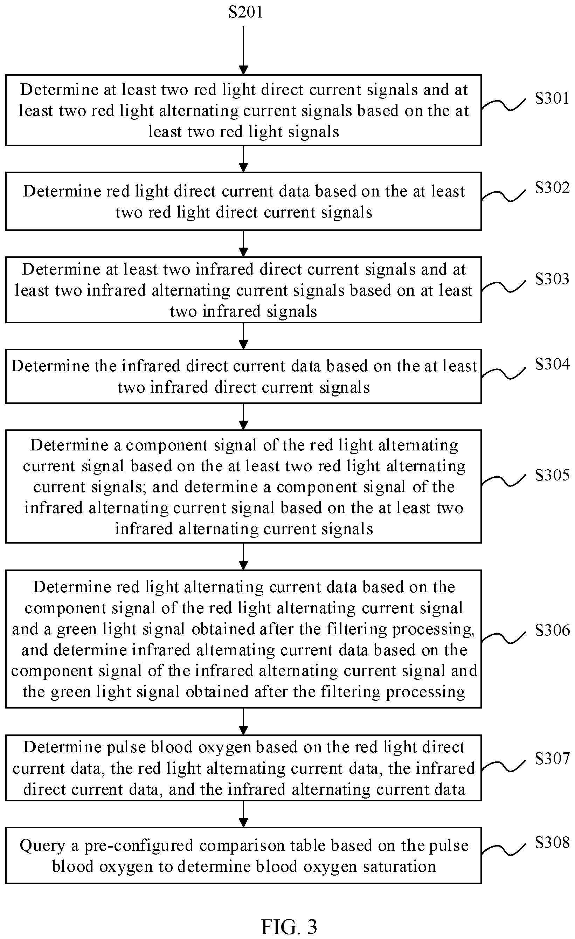

The following more specifically describes the technical solutions of this application with reference to embodiments. FIG. 3 is a flowchart of another blood oxygen detection method according to an embodiment of this application.

As shown in FIG. 3, after S201, the method further includes the following steps.

S301: Determine at least two red light direct current signals and at least two red light alternating current signals based on the at least two red light signals.

Alternating current/direct current separation is performed on red light signals received by a plurality of detection points, to obtain a plurality of red light direct current signals and a plurality of red light alternating current signals. In an example, alternating current/direct current separation is performed on a red light signal received at each detection point, and a red light direct current signal and a red light alternating current signal are obtained at each detection point. In another example, detection may be further performed on each detection point a plurality of times. In each time of detection, alternating current/direct current separation is performed on a red light signal received at each detection point, and a red light direct current signal and a red light alternating current signal are obtained at each detection point.

S302: Determine red light direct current data based on the at least two red light direct current signals.

In an example, one piece of red light direct current data is determined based on a plurality of red light direct current signals determined by a plurality of detection points. In another example, one piece of red light direct current data may be determined based on a plurality of red light direct current signals determined through a plurality of times of measurement at each detection point. The red light direct current data may be an average value, a maximum value, a minimum value, a median, or the like of the plurality of red light direct current signals. A person skilled in the art should note that the direct current data reflects a status of deoxyhemoglobin in a venous blood vessel. Because a blood flow in the venous blood vessel is relatively constant, a plurality of direct current signals may be combined into the red light direct current data, and any equivalent variation or replacement shall fall within the protection scope of this application.

S303: Determine at least two infrared direct current signals and at least two infrared alternating current signals based on the at least two infrared signals.

Alternating current/direct current separation is performed on infrared signals received by a plurality of detection points, to obtain a plurality of infrared direct current signals and a plurality of infrared alternating current signals. In an example, alternating current/direct current separation is performed on an infrared signal received at each detection point, and an infrared direct current signal and an infrared alternating current signal are obtained at each detection point. In another example, detection may be performed on each detection point a plurality of times. In each time of detection, alternating current/direct current separation is performed on an infrared signal received at each detection point, and an infrared direct current signal and an infrared alternating current signal are obtained at each detection point.

S304: Determine the infrared direct current data based on the at least two infrared direct current signals.

In an example, one piece of infrared direct current data is determined based on a plurality of infrared direct current signals determined by a plurality of detection points. In another example, one piece of infrared direct current data may be determined based on a plurality of infrared direct current signals determined through a plurality of times of measurement at each detection point. The infrared direct current data may be an average value, a maximum value, a minimum value, a median, or the like of the plurality of infrared direct current signals. A person skilled in the art should note that the direct current data reflects a status of oxyhemoglobin in a venous blood vessel. Because a blood flow in the venous blood vessel is relatively constant, a plurality of direct current signals may be combined into the red light direct current data, and any equivalent variation or replacement shall fall within the protection scope of this application.

S305: Determine a component signal of the red light alternating current signal based on the at least two red light alternating current signals; and determine a component signal of the infrared alternating current signal based on the at least two infrared alternating current signals.

In an example, a plurality of component signals in the plurality of red light alternating current signals are separated by using the independent component analysis algorithm, to obtain the plurality of component signals of the red light alternating current signals, where the plurality of component signals of the red light alternating current signals include an arterial signal. In addition, a plurality of component signals of the plurality of infrared alternating current signals are separated by using the independent component analysis algorithm, to obtain the plurality of component signals of the infrared alternating current signals, where the plurality of component signals of the infrared alternating current signals include an arterial signal. The plurality of component signals are independent of each other. In other words, the plurality of component signals are irrelevant to each other. In an example, n may be set to a quantity of red light alternating current signals, where n is greater than or equal to 1, and m is set to a quantity of component signals of the red light alternating current signals, where m is greater than or equal to 1. In an embodiment, if n = m, it means that a quantity of red light alternating current signals is equal to a quantity of component signals separated from the red light alternating current signals. The component signals include an arterial signal. Similarly, component signals of the plurality of infrared alternating current signals may be determined. In another example, a plurality of component signals in the plurality of red light/infrared alternating current signals may be separated by using a principal component analysis algorithm, to obtain the plurality of component signals of the red light/infrared alternating current signals. A person skilled in the art should note that, for a method for separating a plurality of component signals from a plurality of alternating current signals, any other equivalent variation or replacement that can be easily figured out shall fall within the protection scope of this application.

S306: Determine red light alternating current data based on the component signal of the red light alternating current signal and a green light signal obtained after filtering processing, and determine infrared alternating current data based on the component signal of the infrared alternating current signal and the green light signal obtained after the filtering processing.

In an example, pre-processing of filtering and denoising is performed on the collected green light signal. Then, a green light signal obtained after the denoising is used as reference data of a pulse signal. The pulse signal is selected from the plurality of determined component signals of the red light alternating current signals, and the pulse signal is used as the red light alternating current data. In an example, linear correlation may be used to determine a correlation degree between the plurality of component signals of the red light alternating current signals and the green light signal obtained after the filtering processing. The correlation degree may be understood as a similarity degree. Then, a component signal that is of a red light alternating current signal and that is most closely correlated with the green light signal obtained after the filtering processing is determined as the red light alternating current data. Likewise, a component signal that is of an infrared alternating current signal and that is most closely correlated with the green light signal obtained after the filtering processing is determined as the infrared alternating current data.

S307: Determine pulse blood oxygen based on the red light direct current data, the red light alternating current data, the infrared direct current data, and the infrared alternating current data.

In an example, after the red light direct current data, the red light alternating current data, the infrared direct current data, and the infrared alternating current data are determined, the pulse blood oxygen may be determined according to a formula $R = \dfrac{\frac{Red_{ac}}{Red_{dc}}}{\frac{IR_{ac}}{IR_{dc}}}$. A value of R represents a concentration proportional relationship between Hb and HbO2 in blood, $Red_{ac}$ is the red light alternating current data, $Red_{dc}$ is the red light direct current data, $IR_{ac}$ is the infrared alternating current data, and $IR_{dc}$ is the infrared direct current data.

S308: Query a pre-configured comparison table based on the pulse blood oxygen to determine blood oxygen saturation.

An SpO2 value corresponding to a pulse blood oxygen value is queried based on the pre-configured comparison table of pulse blood oxygen and SpO2.

In this application, more information is collected by using a plurality of measurement points, then collected alternating current information is separated by using the independent component analysis algorithm, and denoising is performed by using the green light signal, so that interference caused by venous blood flows, capillaries, and the like can be eliminated, a disadvantage of a low signal-to-noise ratio is made up, and accuracy of a blood oxygen measurement on a wrist is increased.

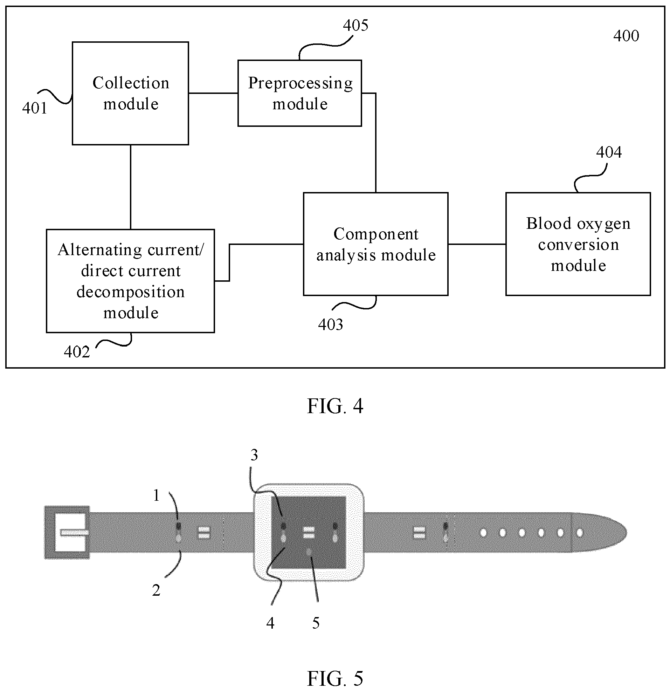

FIG. 4 is a schematic diagram of a blood oxygen detection apparatus according to an embodiment of this application.

As shown in FIG. 4, a blood oxygen detection apparatus 400 is provided. The apparatus 400 includes: a collection module 401, configured to obtain at least two red light signals, at least two infrared signals, and a green light signal; an alternating current/direct current decomposition module 402, configured to: determine red light direct current data and a component signal of a red light alternating current signal based on the at least two red light signals, and determine infrared direct current data and a component signal of an infrared alternating current signal based on the at least two infrared signals, where the component signal includes an arterial signal; a component analysis module 403, configured to: determine red light alternating current data based on the component signal of the red light alternating current signal and the green light signal, and determine infrared alternating current data based on the component signal of the infrared alternating current signal and the green light signal; and a blood oxygen conversion module 404, configured to determine blood oxygen saturation based on the red light direct current data, the red light alternating current data, the infrared direct current data, and the infrared alternating current data.

In a possible implementation, the alternating current/direct current decomposition module 402 is further configured to: determine at least two red light direct current signals and at least two red light alternating current signals based on the at least two red light signals; determine the red light direct current data based on the at least two red light direct current signals; determine at least two infrared direct current signals and at least two infrared alternating current signals based on the at least two infrared signals; determine the infrared direct current data based on the at least two infrared direct current signals; determine the component signal of the red light alternating current signal based on the at least two red light alternating current signals; and determine the component signal of the infrared alternating current signal based on the at least two infrared alternating current signals. In another example, a red light direct current component and an infrared direct current component may alternatively be determined in another independent module.

In a possible implementation, the component analysis module 403 is further configured to: separate at least one component signal of the at least two red light alternating current signals by using an independent component analysis algorithm or a principal component analysis algorithm, to obtain at least one component signal of the red light alternating current signal, where the at least one component signal of the red light alternating current signal includes an arterial signal; and separate at least one component signal of the at least two infrared alternating current signals by using the independent component analysis algorithm or the principal component analysis algorithm, to obtain at least one component signal of the infrared alternating current signal, where the at least one component signal of the infrared alternating current signal includes an arterial signal.

In a possible implementation, the apparatus 400 further includes a preprocessing module 405. The preprocessing module 405 is configured to perform filtering processing on the green light signal. In an example, the preprocessing module may perform filtering processing on the green light signal by using a filter. A person skilled in the art should note that the filter is merely a possible implementation, and any equivalent replacement falls within the protection scope of this application. A specific filter to be used is not limited herein. The component analysis module 403 is further configured to: determine the red light alternating current data based on the component signal of the red light alternating current signal and the green light signal obtained after the filtering processing, and determine the infrared alternating current data based on the component signal of the infrared alternating current signal and the green light signal obtained after the filtering processing.

In a possible implementation, the component analysis module 403 is further configured to: perform comparison based on the at least one component signal of the red light alternating current signal and the green light signal, and determine that data corresponding to at least one component signal, of the red light alternating current signal, closest to the green light signal is the red light alternating current data; and perform comparison based on the at least one component signal of the infrared alternating current signal and the green light signal, and determine that data corresponding to at least one component signal, of the infrared alternating current signal, closest to the green light signal is the infrared alternating current data.

In a possible implementation, the red light direct current data is an average value, a maximum value, a minimum value, or a median of the at least two red light direct current signals; and the infrared direct current data is an average value, a maximum value, a minimum value, or a median of the at least two infrared direct current signals.

In a possible implementation, the blood oxygen conversion module 404 is further configured to: determine pulse blood oxygen based on the red light direct current data, the red light alternating current data, the infrared direct current data, and the infrared alternating current data; and query a pre-configured comparison table based on the pulse blood oxygen, to determine the blood oxygen saturation.

In this application, more information is collected by using a plurality of measurement points, then collected alternating current information is separated by using the independent component analysis algorithm, and denoising is performed by using the green light signal, so that interference caused by venous blood flows, capillaries, and the like can be eliminated, a disadvantage of a low signal-to-noise ratio is made up, and accuracy of a blood oxygen measurement on a wrist is increased.

FIG. 5 is a schematic diagram of another blood oxygen detection apparatus according to an embodiment of this application.

As shown in FIG. 5, a blood oxygen detection apparatus is provided. The apparatus includes at least two red light sensors, at least two infrared sensors, a green light sensor, and a processor. The at least two red light sensors are configured to obtain at least two red light signals, the at least two infrared sensors are configured to obtain at least two infrared signals, and the green light sensor is configured to obtain a green light signal. The processor is configured to: determine red light direct current data and a component signal of a red light alternating current signal based on the at least two red light signals; and determine infrared direct current data and a component signal of an infrared alternating current signal based on the at least two infrared signals. The component signal includes an arterial signal. The processor is further configured to: determine red light alternating current data based on the component signal of the red light alternating current signal and the green light signal; and determine infrared alternating current data based on the component signal of the infrared alternating current signal and the green light signal. The processor is further configured to determine blood oxygen saturation based on the red light direct current data, the red light alternating current data, the infrared direct current data, and the infrared alternating current data.

In an example, the processor is further configured to: determine at least two red light direct current signals and at least two red light alternating current signals based on the at least two red light signals; determine the red light direct current data based on the at least two red light direct current signals; determine at least two infrared direct current signals and at least two infrared alternating current signals based on the at least two infrared signals; determine the infrared direct current data based on the at least two infrared direct current signals; determine the component signal of the red light alternating current signal based on the at least two red light alternating current signals; and determine the component signal of the infrared alternating current signal based on the at least two infrared alternating current signals.

In an example, the processor is further configured to: separate at least one component signal of the at least two red light alternating current signals by using an independent component analysis algorithm or a principal component analysis algorithm, to obtain at least one component signal of the red light alternating current signal, where the at least one component signal of the red light alternating current signal includes an arterial signal; and separate at least one component signal of the at least two infrared alternating current signals by using the independent component analysis algorithm or the principal component analysis algorithm, to obtain at least one component signal of the infrared alternating current signal, where the at least one component signal of the infrared alternating current signal includes an arterial signal.

In an example, the processor is further configured to: perform filtering processing on the green light signal; determine the red light alternating current data based on the component signal of the red light alternating current signal and a green light signal obtained after the filtering processing; and determine the infrared alternating current data based on the component signal of the infrared alternating current signal and the green light signal obtained after the filtering processing.

In an example, the processor is further configured to: perform comparison based on the at least one component signal of the red light alternating current signal and the green light signal, and determine that data corresponding to at least one component signal, of a red light alternating current signal, closest to the green light signal is the red light alternating current data; and perform comparison based on the at least one component signal of the infrared alternating current signal and the green light signal, and determine that data corresponding to at least one component signal, of an infrared alternating current signal, closest to the green light signal is the infrared alternating current data.

In an example, the red light direct current data is an average value, a maximum value, a minimum value, or a median of the at least two red light direct current signals. The infrared direct current data is an average value, a maximum value, a minimum value, or a median of the at least two infrared direct current signals.

In an example, the processor is further configured to: determine pulse blood oxygen based on the red light direct current data, the red light alternating current data, the infrared direct current data, and the infrared alternating current data; and query a pre-configured comparison table based on the pulse blood oxygen, to determine the blood oxygen saturation.

In an example, the apparatus is a wearable intelligent device, and includes a watchband and an intelligent wearable device body. At least one red light sensor and at least one infrared sensor are disposed on the watchband. At least one red light sensor, at least one infrared sensor, and a processor are disposed on the intelligent wearable device body. A green light sensor is disposed on the watchband or the intelligent wearable device body.

In an example, at least two detection points are disposed on the blood oxygen detection apparatus. A red light sensor and an infrared sensor are disposed at each detection point. Arteries at a wrist are mostly distributed in a deep part of a human body, while veins are distributed near a surface of skin. A signal measured on the back of the wrist contains more venous compositions. On both sides of the wrist, arteries near ulna styloid process and radial styloid process are slightly near the skin, and data from these two positions contains less venous compositions. As shown in FIG. 5, the blood oxygen detection apparatus may be in a form of a smart watch, and two detection points 1/2 and 3/4 may be disposed on the smart watch. The detection points 3/4 may be disposed on a back of a watch face of the smart watch. When the smart watch is worn, the detection point 3/4 is disposed on a back of a wrist of a user. The detection point 1/2 may be located within a 1 cm range of the ulna styloid process or radial styloid process. In other words, the detection point 1/2 may be disposed on the watchband. 1 and 3 are red light sensors, and 2 and 4 are infrared sensors. In addition, the apparatus is further provided with a photoplethysmography (photo plethysmo graphy, PPG) sensor, namely, the foregoing green light sensor, which is marked as 5 in FIG. 5.

A person skilled in the art should note that a location of the detection point may be any location on the device. FIG. 5 is merely an optional implementation, and any equivalent replacement falls within the protection scope of this application. A specific location of the detection point is not limited herein. A person skilled in the art should further note that the red light sensor and the infrared sensor at the detection point may be separated, or may be integrated together. A person skilled in the art should further note that a location of the PPG sensor used to detect green light may also be any location on the device. FIG. 5 is merely an optional implementation, and any equivalent replacement falls within the protection scope of this application. A specific location of the PPG sensor is not limited herein.

In this application, more information is collected by using the plurality of measurement points, then a plurality of component signals are separated from collected alternating current information by using the independent component analysis algorithm or the principal component analysis algorithm, and denoising is performed by using the green light signal, so that interference caused by venous blood flows, capillaries, and the like can be eliminated, a disadvantage of a low signal-to-noise ratio can be made up and accuracy of a blood oxygen measurement on the wrist is increased.

An ordinary person in the art may be further aware that, in combination with the examples described in the embodiments disclosed in this specification, units and algorithm steps may be implemented by electronic hardware, computer software, or a combination thereof. To clearly describe the interchangeability between the hardware and the software, the foregoing has generally described compositions and steps of each example according to functions. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

Persons of ordinary skill in the art may understand that all or some of the steps in each of the foregoing method of the embodiments may be implemented by a program instructing a processor. The foregoing program may be stored in a computer-readable storage medium. The storage medium may be a non-transitory (non-transitory) medium, for example may be a random-access memory, read-only memory, a flash memory, a hard disk, a solid state drive, a magnetic tape (magnetic tape), a floppy disk (floppy disk), an optical disc (optical disc), or any combination thereof.

## Claims

1. A blood oxygen detection method performed by a wearable intelligent device that comprises: a watchband; an intelligent wearable device body; at least one red light sensor (1) and at least one infrared sensor (2) disposed on the watchband; at least one red light sensor (3), at least one infrared sensor (4), and a processor disposed on the intelligent wearable device body; and a green light sensor (5) disposed on the watchband or the intelligent wearable device body, wherein the method comprises:

   obtaining (S201) at least two red light signals from the red light sensors (1, 3), at least two infrared signals from the infrared sensors (2, 4), and a green light signal from the green light sensor (5);
   determining (S202) red light direct current data and a component signal of a red light alternating current signal based on the at least two red light signals; and determining infrared direct current data and a component signal of an infrared alternating current signal based on the at least two infrared signals, wherein the component signal comprises an arterial signal;
   determining (S203) red light alternating current data based on the component signal of the red light alternating current signal and the green light signal; and determining infrared alternating current data based on the component signal of the infrared alternating current signal and the green light signal; and
   determining (S204) blood oxygen saturation based on the red light direct current data, the red light alternating current data, the infrared direct current data, and the infrared alternating current data.

2. The method according to claim 1, wherein the determining red light direct current data and a component signal of a red light alternating current signal based on the at least two red light signals; and determining infrared direct current data and a component signal of an infrared alternating current signal based on the at least two infrared signals comprises:

determining (S301) at least two red light direct current signals and at least two red light alternating current signals based on the at least two red light signals;
determining (S302) the red light direct current data based on the at least two red light direct current signals;
determining (S303) at least two infrared direct current signals and at least two infrared alternating current signals based on the at least two infrared signals;
determining (S304) the infrared direct current data based on the at least two infrared direct current signals; and
determining (S305) the component signal of the red light alternating current signal based on the at least two red light alternating current signals, and determining the component signal of the infrared alternating current signal based on the at least two infrared alternating current signals.

3. The method according to claim 2, wherein the determining the component signal of the red light alternating current signal based on the at least two red light alternating current signals, and determining the component signal of the infrared alternating current signal based on the at least two infrared alternating current signals comprises:

separating at least one component signal of the at least two red light alternating current signals by using an independent component analysis algorithm or a principal component analysis algorithm, to obtain at least one component signal of the red light alternating current signal, wherein the at least one component signal of the red light alternating current signal comprises an arterial signal; and
separating at least one component signal of the at least two infrared alternating current signals by using the independent component analysis algorithm or the principal component analysis algorithm, to obtain at least one component signal of the infrared alternating current signal, wherein the at least one component signal of the infrared alternating current signal comprises an arterial signal.

4. The method according to any one of claims 1 to 3, wherein the determining red light alternating current data based on the component signal of the red light alternating current signal and the green light signal;

and determining infrared alternating current data based on the component signal of the infrared alternating current signal and the green light signal comprises:

performing filtering processing on the green light signal; and
determining the red light alternating current data based on the component signal of the red light alternating current signal and a green light signal obtained after the filtering processing, and determining the infrared alternating current data based on the component signal of the infrared alternating current signal and the green light signal obtained after the filtering processing.

5. The method according to claim 3 or 4, wherein the determining red light alternating current data based on the component signal of the red light alternating current signal and the green light signal; and determining infrared alternating current data based on the component signal of the infrared alternating current signal and the green light signal comprises:

performing comparison based on the at least one component signal of the red light alternating current signal and the green light signal, and determining that data corresponding to at least one component signal, of the red light alternating current signal, closest to the green light signal is the red light alternating current data; and
performing comparison based on the at least one component signal of the infrared alternating current signal and the green light signal, and determining that data corresponding to at least one component signal, of the infrared alternating current signal, closest to the green light signal is the infrared alternating current data.

6. The method according to any one of claims 1 to 5, wherein

the red light direct current data is an average value, a maximum value, a minimum value, or a median of the at least two red light direct current signals; and
the infrared direct current data is an average value, a maximum value, a minimum value, or a median of the at least two infrared direct current signals.

7. The method according to any one of claims 1 to 6, wherein the determining blood oxygen saturation based on the red light direct current data, the red light alternating current data, the infrared direct current data, and the infrared alternating current data comprises:

determining (S307) pulse blood oxygen based on the red light direct current data, the red light alternating current data, the infrared direct current data, and the infrared alternating current data; and

querying (S308) a pre-configured comparison table based on the pulse blood oxygen, to determine the blood oxygen saturation.

8. A blood oxygen detection apparatus, wherein the apparatus is a wearable intelligent device that comprises: a watchband; an intelligent wearable device body; at least one red light sensor (1) and at least one infrared sensor (2) disposed on the watchband; at least one red light sensor (3), at least one infrared sensor (4), and a processor disposed on the intelligent wearable device body; and a green light sensor (5) disposed on the watchband or the intelligent wearable device body, wherein

the processor is configured to: obtain (S201) at least two red light signals from the red light sensors (1, 3), obtain at least two infrared signals from the infrared sensors (2, 4), and obtain a green light signal from the green light sensor (5);

the processor is configured to: determine (S202) red light direct current data and a component signal of a red light alternating current signal based on the at least two red light signals; and determine infrared direct current data and a component signal of an infrared alternating current signal based on the at least two infrared signals, wherein the component signal comprises an arterial signal;

the processor is further configured to: determine (S203) red light alternating current data based on the component signal of the red light alternating current signal and the green light signal; and determine infrared alternating current data based on the component signal of the infrared alternating current signal and the green light signal; and

the processor is further configured to determine (S204) blood oxygen saturation based on the red light direct current data, the red light alternating current data, the infrared direct current data, and the infrared alternating current data.

9. The apparatus according to claim 8, wherein the processor is further configured to:

determine (S301) at least two red light direct current signals and at least two red light alternating current signals based on the at least two red light signals;

determine (S302) the red light direct current data based on the at least two red light direct current signals;

determine at least two infrared direct current signals and at least two infrared alternating current signals based on the at least two infrared signals;

determine (S303) the infrared direct current data based on the at least two infrared direct current signals; and

determine (S304) the component signal of the red light alternating current signal based on the at least two red light alternating current signals; and determine the component signal of the infrared alternating current signal based on the at least two infrared alternating current signals.

10. The apparatus according to claim 9, wherein the processor is further configured to:

separate at least one component signal of the at least two red light alternating current signals by using an independent component analysis algorithm or a principal component analysis algorithm, to obtain at least one component signal of the red light alternating current signal, wherein the at least one component signal of the red light alternating current signal comprises an arterial signal; and

separate at least one component signal of the at least two infrared alternating current signals by using the independent component analysis algorithm or the principal component analysis algorithm, to obtain at least one component signal of the infrared alternating current signal, wherein the at least one component signal of the infrared alternating current signal comprises an arterial signal.

11. The apparatus according to any one of claim 8 to 10, wherein the processor is further configured to:

perform filtering processing on the green light signal; and

determine the red light alternating current data based on the component signal of the red light alternating current signal and a green light signal obtained after the filtering processing, and determine the infrared alternating current data based on the component signal of the infrared alternating current signal and the green light signal obtained after the filtering processing.

12. The apparatus according to claim 10 or 11, wherein the processor is further configured to:

perform comparison based on the at least one component signal of the red light alternating current signal and the green light signal, and determine that data corresponding to at least one component signal, of the red light alternating current signal, closest to the green light signal is the red light alternating current data; and

perform comparison based on the at least one component signal of the infrared alternating current signal and the green light signal, and determine that data corresponding to at least one component signal, of the infrared alternating current signal, closest to the green light signal is the infrared alternating current data.

13. The apparatus according to any one of claims 8 to 12, wherein

the red light direct current data is an average value, a maximum value, a minimum value, or a median of the at least two red light direct current signals; and the infrared direct current data is an average value, a maximum value, a minimum value, or a median of the at least two infrared direct current signals.

14. The apparatus according to any one of claims 8 to 13, wherein the processor is further configured to:

determine pulse blood oxygen based on the red light direct current data, the red light alternating current data, the infrared direct current data, and the infrared alternating current data; and query a pre-configured comparison table based on the pulse blood oxygen, to determine the blood oxygen saturation.

**Patentansprüche**

1. Blutsauerstoffdetektionsverfahren, das durch eine tragbare intelligente Vorrichtung durchgeführt wird, die Folgendes umfasst: ein Uhrband; einen Körper einer intelligenten tragbaren Vorrichtung; mindestens einen Rotlichtsensor (1) und mindestens einen Infrarotsensor (2), die an dem Uhrband angeordnet sind; mindestens einen Rotlichtsensor (3), mindestens einen Infrarotsensor (4) und einen Prozessor, die an dem Körper der intelligenten tragbaren Vorrichtung angeordnet sind; und einen Grünlichtsensor (5), der an dem Uhrband oder dem Körper der intelligenten tragbaren Vorrichtung angeordnet ist, wobei das Verfahren Folgendes umfasst:

Erlangen (S201) von mindestens zwei Rotlichtsignalen von den Rotlichtsensoren (1, 3), mindestens zwei Infrarotsignalen von den Infrarotsensoren (2, 4) und eines Grünlichtsignals von dem Grünlichtsensor (5); Bestimmen (S202) von Rotlicht-Gleichstromdaten und eines Komponentensignals eines Rotlicht-Wechselstromsignals basierend auf den mindestens zwei Rotlichtsignalen; und Bestimmen von Infrarot-Gleichstromdaten und eines Komponentensignals eines Infrarot-Wechselstromsignals basierend auf den mindestens zwei Infrarotsignalen, wobei das Komponentensignal ein arterielles Signal umfasst; Bestimmen (S203) von Rotlicht-Wechselstromdaten basierend auf dem Komponentensignal des Rotlicht-Wechselstromsignals und dem Grünlichtsignal; und Bestimmen von Infrarot-Wechselstromdaten basierend auf dem Komponentensignal des Infrarot-Wechselstromsignals und dem Grünlichtsignal; und Bestimmen (S204) der Blutsauerstoffsättigung basierend auf den Rotlicht-Gleichstromdaten, den Rotlicht-Wechselstromdaten, den Infrarot-Gleichstromdaten und den Infrarot-Wechselstromdaten.

2. Verfahren nach Anspruch 1, wobei das Bestimmen von Rotlicht-Gleichstromdaten und eines Komponentensignals eines Rotlicht-Wechselstromsignals basierend auf den mindestens zwei Rotlichtsignalen; und das Bestimmen von Infrarot-Gleichstromdaten und eines Komponentensignals eines Infrarot-Wechselstromsignals basierend auf den mindestens zwei Infrarotsignalen Folgendes umfasst:

Bestimmen (S301) von mindestens zwei Rotlicht-Gleichstromsignalen und mindestens zwei Rotlicht-Wechselstromsignalen basierend auf den mindestens zwei Rotlichtsignalen; Bestimmen (S302) der Rotlicht-Gleichstromdaten basierend auf den mindestens zwei Rotlicht-Gleichstromsignalen; Bestimmen (S303) von mindestens zwei Infrarot-Gleichstromsignalen und mindestens zwei Infrarot-Wechselstromsignalen basierend auf den mindestens zwei Infrarotsignalen; Bestimmen (S304) der Infrarot-Gleichstromdaten basierend auf den mindestens zwei Infrarot-Gleichstromsignalen; und Bestimmen (S305) des Komponentensignals des Rotlicht-Wechselstromsignals basierend auf den mindestens zwei Rotlicht-Wechselstromsignalen und Bestimmen des Komponentensignals des Infrarot-Wechselstromsignals basierend auf den mindestens zwei Infrarot-Wechselstromsignalen.

3. Verfahren nach Anspruch 2, wobei das Bestimmen des Komponentensignals des Rotlicht-Wechselstromsignals basierend auf den mindestens zwei Rotlicht-Wechselstromsignalen und das Bestimmen des Komponentensignals des Infrarot-Wechselstromsignals basierend auf den mindestens zwei Infrarot-Wechselstromsignalen Folgendes umfasst:

Abtrennen mindestens eines Komponentensignals der mindestens zwei Rotlicht-Wechselstromsignale unter Verwendung eines unabhängigen Komponentenanalysealgorithmus oder eines Komponentenanalysehauptalgorithmus, um mindestens ein Komponentensignal des Rotlicht-Wechselstromsignals zu erlangen, wobei das mindestens eine Komponentensignal des Rotlicht-Wechselstromsignals ein arterielles Signal umfasst; und

Abtrennen mindestens eines Komponentensignals der mindestens zwei Infrarot-Wechselstromsignale unter Verwendung des unabhängigen Komponentenanalysealgorithmus oder des Komponentenanalysehauptalgorithmus, um mindestens ein Komponentensignal des Infrarot-Wechselstromsignals zu erlangen, wobei das mindestens eine Komponentensignal des Infrarot-Wechselstromsignals ein arterielles Signal umfasst.

4. Verfahren nach einem der Ansprüche 1 bis 3, wobei das Bestimmen von Rotlicht-Wechselstromdaten basierend auf dem Komponentensignal des Rotlicht-Wechselstromsignals und dem Grünlichtsignal; und das Bestimmen von Infrarot-Wechselstromdaten basierend auf dem Komponentensignal des Infrarot-Wechselstromsignals und dem Grünlichtsignal Folgendes umfasst:

Durchführen von Filterverarbeitung an dem Grünlichtsignal; und

Bestimmen der Rotlicht-Wechselstromdaten basierend auf dem Komponentensignal des Rotlicht-Wechselstromsignals und einem Grünlichtsignal, das nach der Filterverarbeitung erlangt wird, und Bestimmen der Infrarot-Wechselstromdaten basierend auf dem Komponentensignal des Infrarot-Wechselstromsignals und dem Grünlichtsignal, das nach der Filterverarbeitung erlangt wird.

5. Verfahren nach einem der Ansprüche 3 oder 4, wobei das Bestimmen von Rotlicht-Wechselstromdaten basierend auf dem Komponentensignal des Rotlicht-Wechselstromsignals und dem Grünlichtsignal; und das Bestimmen von Infrarot-Wechselstromdaten basierend auf dem Komponentensignal des Infrarot-Wechselstromsignals und dem Grünlichtsignal Folgendes umfasst:

Durchführen eines Vergleichs basierend auf dem mindestens einen Komponentensignal des Rotlicht-Wechselstromsignals und dem Grünlichtsignal, und Bestimmen, dass Daten, die mindestens einem Komponentensignal des Rotlicht-Wechselstromsignals entsprechen, das dem Grünlichtsignal am nächsten liegt, die Rotlicht-Wechselstromdaten sind; und Durchführen eines Vergleichs basierend auf dem mindestens einen Komponentensignal des Infrarot-Wechselstromsignals und dem Grünlichtsignal, und Bestimmen, dass Daten, die mindestens einem Komponentensignal des Infrarot-Wechselstromsignals entsprechen, das dem Grünlichtsignal am nächsten liegt, die Infrarot-Wechselstromdaten sind.

6. Verfahren nach einem der Ansprüche 1 bis 5, wobei

die Rotlicht-Gleichstromdaten ein Mittelwert, ein Maximalwert, ein Minimalwert oder ein Median der mindestens zwei Rotlicht-Gleichstromsignale sind; und

die Infrarot-Gleichstromdaten ein Mittelwert, ein Maximalwert, ein Minimalwert oder ein Median der mindestens zwei Infrarot-Gleichstromsignale sind.

7. Verfahren nach einem der Ansprüche 1 bis 6, wobei das Bestimmen der Blutsauerstoffsättigung basierend auf den Rotlicht-Gleichstromdaten, den Rotlicht-Wechselstromdaten, den Infrarot-Gleichstromdaten und den Infrarot-Wechselstromdaten Folgendes umfasst:

Bestimmen (S307) von Pulsblutsauerstoff basierend auf den Rotlicht-Gleichstromdaten, den Rotlicht-Wechselstromdaten, den Infrarot-Gleichstromdaten und den Infrarot-Wechselstromdaten; und

Abfragen (S308) einer vorkonfigurierten Vergleichstabelle basierend auf dem Pulsblutsauerstoff, um die Blutsauerstoffsättigung zu bestimmen.

8. Blutsauerstoffdetektionsgerät, wobei das Gerät eine tragbare intelligente Vorrichtung ist, die Folgendes umfasst: ein Uhrband; einen Körper einer intelligenten tragbaren Vorrichtung; mindestens einen Rotlichtsensor (1) und mindestens einen Infrarotsensor (2), die an dem Uhrband angeordnet sind; mindestens einen Rotlichtsensor (3), mindestens einen Infrarotsensor (4) und einen Prozessor, die an dem Körper der intelligenten tragbaren Vorrichtung angeordnet sind; und einen Grünlichtsensor (5), der an dem Uhrband oder dem Körper der intelligenten tragbaren Vorrichtung angeordnet ist, wobei der Prozessor zu Folgendem konfiguriert ist: Erlangen (S201) von mindestens zwei Rotlichtsignalen von den Rotlichtsensoren (1, 3), Erlangen von mindestens zwei Infrarotsignalen von den Infrarotsensoren (2, 4) und Erlangen eines Grünlichtsignals von dem Grünlichtsensor (5);

der Prozessor zu Folgendem konfiguriert ist: Bestimmen (S202) von Rotlicht-Gleichstromdaten und eines Komponentensignals eines Rotlicht-Wechselstromsignals basierend auf den mindestens zwei Rotlichtsignalen; und Bestimmen von Infrarot-Gleichstromdaten und eines Komponentensignals eines Infrarot-Wechselstromsignals basierend auf den mindestens zwei Infrarotsignalen, wobei das Komponentensignal ein arterielles Signal umfasst;

der Prozessor ferner zu Folgendem konfiguriert ist: Bestimmen (S203) von Rotlicht-Wechselstromdaten basierend auf dem Komponentensignal des Rotlicht-Wechselstromsignals und dem Grünlichtsignal; und Bestimmen von Infrarot-Wechselstromdaten basierend auf dem Komponentensignal des Infrarot-Wechselstromsignals und dem Grünlichtsignal; und

der Prozessor ferner zu Folgendem konfiguriert ist: Bestimmen (S204) der Blutsauerstoffsättigung basierend auf den Rotlicht-Gleichstromdaten, den Rotlicht-Wechselstromdaten, den Infrarot-Gleichstromdaten und den Infrarot-Wechselstromdaten.

9. Gerät nach Anspruch 8, wobei der Prozessor ferner zu Folgendem konfiguriert ist:

Bestimmen (S301) von mindestens zwei Rotlicht-Gleichstromsignalen und mindestens zwei Rotlicht-Wechselstromsignalen basierend auf den mindestens zwei Rotlichtsignalen;
Bestimmen (S302) der Rotlicht-Gleichstromdaten basierend auf den mindestens zwei Rotlicht-Gleichstromsignalen;
Bestimmen von mindestens zwei Infrarot-Gleichstromsignalen und mindestens zwei Infrarot-Wechselstromsignalen basierend auf den mindestens zwei Infrarotsignalen;
Bestimmen (S303) der Infrarot-Gleichstromdaten basierend auf den mindestens zwei Infrarot-Gleichstromsignalen; und
Bestimmen (S304) des Komponentensignals des Rotlicht-Wechselstromsignals basierend auf den mindestens zwei Rotlicht-Wechselstromsignalen; und Bestimmen des Komponentensignals des Infrarot-Wechselstromsignals basierend auf den mindestens zwei Infrarot-Wechselstromsignalen.

10. Gerät nach Anspruch 9, wobei der Prozessor ferner zu Folgendem konfiguriert ist:

Abtrennen mindestens eines Komponentensignals der mindestens zwei Rotlicht-Wechselstromsignale unter Verwendung eines unabhängigen Komponentenanalysealgorithmus oder eines Komponentenanalysehauptalgorithmus, um mindestens ein Komponentensignal des Rotlicht-Wechselstromsignals zu erlangen, wobei das mindestens eine Komponentensignal des Rotlicht-Wechselstromsignals ein arterielles Signal umfasst; und
Abtrennen mindestens eines Komponentensignals der mindestens zwei Infrarot-Wechselstromsignale unter Verwendung des unabhängigen Komponentenanalysealgorithmus oder des Komponentenanalysehauptalgorithmus, um mindestens ein Komponentensignal des Infrarot-Wechselstromsignals zu erlangen, wobei das mindestens eine Komponentensignal des Infrarot-Wechselstromsignals ein arterielles Signal umfasst.

11. Gerät nach einem der Ansprüche 8 bis 10, wobei der Prozessor ferner zu Folgendem konfiguriert ist:

Durchführen von Filterverarbeitung an dem Grünlichtsignal; und
Bestimmen der Rotlicht-Wechselstromdaten basierend auf dem Komponentensignal des Rotlicht-Wechselstromsignals und einem Grünlichtsignal, das nach der Filterverarbeitung erlangt wird, und Bestimmen der Infrarot-Wechselstromdaten basierend auf dem Komponentensignal des Infrarot-Wechselstromsignals und dem Grünlichtsignal, das nach der Filterverarbeitung erlangt wird.

12. Gerät nach Anspruch 10 oder 11, wobei der Prozessor ferner zu Folgendem konfiguriert ist:

Durchführen eines Vergleichs basierend auf dem mindestens einen Komponentensignal des Rotlicht-Wechselstromsignals und dem Grünlichtsignal, und Bestimmen, dass Daten, die mindestens einem Komponentensignal des Rotlicht-Wechselstromsignals entsprechen, das dem Grünlichtsignal am nächsten liegt, die Rotlicht-Wechselstromdaten sind; und
Durchführen eines Vergleichs basierend auf dem mindestens einen Komponentensignal des Infrarot-Wechselstromsignals und dem Grünlichtsignal, und Bestimmen, dass Daten, die mindestens einem Komponentensignal des Infrarot-Wechselstromsignals entsprechen, das dem Grünlichtsignal am nächsten liegt, die Infrarot-Wechselstromdaten sind.

13. Gerät nach einem der Ansprüche 8 bis 12, wobei

die Rotlicht-Gleichstromdaten ein Mittelwert, ein Maximalwert, ein Minimalwert oder ein Median der mindestens zwei Rotlicht-Gleichstromsignale sind; und
die Infrarot-Gleichstromdaten ein Mittelwert, ein Maximalwert, ein Minimalwert oder ein Median der mindestens zwei Infrarot-Gleichstromsignale sind.

14. Gerät nach einem der Ansprüche 8 bis 13, wobei der Prozessor ferner zu Folgendem konfiguriert ist:

Bestimmen von Pulsblutsauerstoff basierend auf den Rotlicht-Gleichstromdaten, den Rotlicht-Wechselstromdaten, den Infrarot-Gleichstromdaten und den Infrarot-Wechselstromdaten; und
Abfragen einer vorkonfigurierten Vergleichstabelle basierend auf dem Pulsblutsauerstoff, um die Blutsauerstoffsättigung zu bestimmen.

## Revendications

1. Procédé de détection d'oxygène dans le sang réalisé par un dispositif intelligent portable qui comprend : un bracelet de montre ; un corps de dispositif portable intelligent ; au moins un capteur de lumière rouge (1) et au moins un capteur infrarouge (2) disposés sur le bracelet de montre ; au moins un capteur de lumière rouge (3), au moins un capteur infrarouge (4), et un processeur disposés sur le corps de dispositif portable intelligent ; et un capteur de lumière verte (5) disposé sur le bracelet de montre ou le corps de dispositif portable intelligent, dans lequel le procédé comprend :

l'obtention (S201) d'au moins deux signaux de lumière rouge à partir des capteurs de lumière rouge (1, 3), d'au moins deux signaux infrarouges à partir des capteurs infrarouges (2, 4), et d'un signal de lumière verte à partir du capteur de lumière verte (5) ;
la détermination (S202) de données de courant continu de lumière rouge et d'un signal de composante d'un signal de courant alternatif de lumière rouge sur la base des au moins deux signaux de lumière rouge ; et la détermination de données de courant continu infrarouge et d'un signal de composante d'un signal de courant alternatif infrarouge sur la base de l'au moins deux signaux infrarouges, dans lequel le signal de composante comprend un signal artériel ;
la détermination (S203) de données de courant alternatif de lumière rouge sur la base du signal de composante du signal de courant alternatif de lumière rouge et du signal de lumière verte ; et la détermination de données de courant alternatif infrarouge sur la base du signal de composante du signal de courant alternatif infrarouge et du signal de lumière verte ; et
la détermination (S204) de la saturation en oxygène du sang sur la base des données de courant continu de lumière rouge, des données de courant alternatif de lumière rouge, des données de courant continu infrarouge et des données de courant alternatif infrarouge.

2. Procédé selon la revendication 1, dans lequel la détermination de données de courant continu de lumière rouge et d'un signal de composante d'un signal de courant alternatif de lumière rouge sur la base des au moins deux signaux de lumière rouge ; et la détermination de données de courant continu infrarouge et d'un signal de composante d'un signal de courant alternatif infrarouge sur la base des au moins deux signaux infrarouges comprend :

la détermination (S301) d'au moins deux signaux de courant continu de lumière rouge et d'au moins deux signaux de courant alternatif de lumière rouge sur la base des au moins deux signaux de lumière rouge ;
la détermination (S302) des données de courant continu de lumière rouge sur la base des au moins deux signaux de courant continu de lumière rouge ;
la détermination (S303) d'au moins deux signaux de courant continu infrarouge et d'au moins deux signaux de courant alternatif infrarouge sur la base des au moins deux signaux infrarouges ;
la détermination (S304) des données de courant continu infrarouge sur la base des au moins deux signaux de courant continu infrarouge ; et
la détermination (S305) du signal de composante du signal de courant alternatif de lumière rouge sur la base des au moins deux signaux de courant alternatif de lumière rouge, et la détermination du signal de composante du signal de courant alternatif infrarouge sur la base des au moins deux signaux de courant alternatif infrarouge.

3. Procédé selon la revendication 2, dans lequel la détermination du signal de composante du signal de courant alternatif de lumière rouge sur la base des au moins deux signaux de courant alternatif de lumière rouge, et la détermination du signal de composante du signal de courant alternatif infrarouge sur la base des au moins deux signaux de courant alternatif infrarouge comprend :

la séparation d'au moins un signal de composante des au moins deux signaux de courant alternatif de lumière rouge en utilisant un algorithme d'analyse en composantes indépendantes ou d'un algorithme d'analyse en composantes principales, pour obtenir au moins un signal de composante du signal de courant alternatif de lumière rouge, dans lequel l'au moins un signal de composante du signal de courant alternatif de lumière rouge comprend un signal artériel ; et

la séparation d'au moins un signal de composante des au moins deux signaux de courant alternatif infrarouge en utilisant l'algorithme d'analyse en composantes indépendantes ou l'algorithme d'analyse en composantes principales, pour obtenir au moins un signal de composante du signal de courant alternatif infrarouge, dans lequel l'au moins un signal de composante du signal de courant alternatif infrarouge comprend un signal artériel.

4. Procédé selon l'une quelconque des revendications 1 à 3, dans lequel la détermination de données de courant alternatif de lumière rouge sur la base du signal de composante du signal de courant alternatif de lumière rouge et du signal de lumière verte ; et la détermination de données de courant alternatif infrarouge sur la base du signal de composante du signal de courant alternatif infrarouge et du signal de lumière verte comprend :

la réalisation d'un traitement de filtrage sur le signal de lumière verte ; et

la détermination des données de courant alternatif de lumière rouge sur la base du signal de composante du signal de courant alternatif de lumière rouge et d'un signal de lumière verte obtenu après le traitement de filtrage, et la détermination des données de courant alternatif infrarouge sur la base du signal de composante du signal de courant alternatif infrarouge et du signal de lumière verte obtenu après le traitement de filtrage.

5. Procédé selon la revendication 3 ou 4, dans lequel la détermination de données de courant alternatif de lumière rouge sur la base du signal de composante du signal de courant alternatif de lumière rouge et du signal de lumière verte ; et la détermination de données de courant alternatif infrarouge sur la base du signal de composante du signal de courant alternatif infrarouge et du signal de lumière verte comprend :

la réalisation d'une comparaison sur la base de l'au moins un signal de composante du signal de courant alternatif de lumière rouge et du signal de lumière verte, et la détermination du fait que des données correspondant à au moins un signal de composante, du signal de courant alternatif de lumière rouge, le plus proche du signal de lumière verte, sont les données de courant alternatif de lumière rouge ; et la réalisation d'une comparaison sur la base de l'au moins un signal de composante du signal de courant alternatif infrarouge et du signal de lumière verte, et la détermination du fait que des données correspondant à au moins un signal de composante, du signal de courant alternatif infrarouge, le plus proche du signal de lumière verte sont les données de courant alternatif infrarouge.

6. Procédé selon l'une quelconque des revendications 1 à 5, dans lequel

les données de courant continu de lumière rouge sont une valeur moyenne, une valeur maximale, une valeur minimale, ou une médiane des au moins deux signaux de courant continu de lumière rouge ; et les données de courant continu infrarouge sont une valeur moyenne, une valeur maximale, une valeur minimale ou une médiane des au moins deux signaux de courant continu infrarouge.

7. Procédé selon l'une quelconque des revendications 1 à 6, dans lequel la détermination de la saturation en oxygène du sang sur la base des données de courant continu de lumière rouge, des données de courant alternatif de lumière rouge, des données de courant continu infrarouge et des données de courant alternatif infrarouge comprend :

la détermination (S307) de l'oxygène du sang pulsé sur la base des données de courant continu de lumière rouge, des données de courant alternatif de lumière rouge, des données de courant continu infrarouge et des données de courant alternatif infrarouge ; et l'interrogation (S308) d'une table de comparaison préconfigurée sur la base de l'oxygène du sang pulsé pour déterminer la saturation en oxygène du sang.

8. Appareil de détection d'oxygène dans le sang, dans lequel l'appareil est un dispositif intelligent portable qui comprend : un bracelet de montre ; un corps de dispositif portable intelligent ; au moins un capteur de lumière rouge (1) et au moins un capteur infrarouge (2) disposés sur le bracelet de montre ; au moins un capteur de lumière rouge (3), au moins un capteur infrarouge (4) et un processeur disposés sur le corps de dispositif portable intelligent ; et un capteur de lumière verte (5) disposé sur le bracelet de montre ou le corps de dispositif portable intelligent, dans lequel le processeur est configuré pour : obtenir (S201) au moins deux signaux de lumière rouge à partir des capteurs de lumière rouge (1, 3), obtenir au moins deux signaux infrarouges à partir des capteurs infrarouges (2, 4), et obtenir un signal de lumière verte à partir du capteur de lumière verte (5) ;

le processeur est configuré pour : déterminer (S202) des données de courant continu de lumière rouge et un signal de composante d'un signal de courant alternatif de lumière rouge sur la base des au moins deux signaux de lumière rouge ; et déterminer des données de courant continu infrarouge et un signal de composante d'un signal de courant alternatif infrarouge sur la base des au moins deux signaux infrarouges, dans lequel le signal de composante comprend un signal artériel ;

le processeur est également configuré pour : déterminer (S203) des données de courant alternatif de lumière rouge sur la base du signal de composante du signal de courant alternatif de lumière rouge et du signal de lumière verte ; et déterminer des données de courant alternatif infrarouge sur la base du signal de composante du signal de courant alternatif infrarouge et du signal de lumière verte ; et

le processeur est également configuré pour déterminer (S204) la saturation en oxygène du sang sur la base des données de courant continu de lumière rouge, des données de courant alternatif de lumière rouge, des données de courant continu infrarouge et des données de courant alternatif infrarouge.

9. Appareil selon la revendication 8, dans lequel le processeur est également configuré pour :

déterminer (S301) au moins deux signaux de courant continu de lumière rouge et au moins deux signaux de courant alternatif de lumière rouge sur la base des au moins deux signaux de lumière rouge ;

déterminer (S302) les données de courant continu de lumière rouge sur la base des au moins deux signaux de courant continu de lumière rouge ;

déterminer au moins deux signaux de courant continu infrarouge et au moins deux signaux de courant alternatif infrarouge sur la base des au moins deux signaux infrarouges ;

déterminer (S303) les données de courant continu infrarouge sur la base des au moins deux signaux de courant continu infrarouge ; et

déterminer (S304) le signal de composante du signal de courant alternatif de lumière rouge sur la base des au moins deux signaux de courant alternatif de lumière rouge ; et déterminer le signal de composante du signal de courant alternatif infrarouge sur la base des au moins deux signaux de courant alternatif infrarouge.

10. Appareil selon la revendication 9, dans lequel le processeur est également configuré pour :

séparer au moins un signal de composante des au moins deux signaux de courant alternatif de lumière rouge en utilisant un algorithme d'analyse en composantes indépendantes ou un algorithme d'analyse en composantes principales, pour obtenir au moins un signal de composante du signal de courant alternatif de lumière rouge, dans lequel l'au moins un signal de composante du signal de courant alternatif de lumière rouge comprend un signal artériel ; et

séparer au moins un signal de composante des au moins deux signaux de courant alternatif infrarouge en utilisant l'algorithme d'analyse en composantes indépendantes ou l'algorithme d'analyse en composantes principales, pour obtenir au moins un signal de composante du signal de courant alternatif infrarouge, dans lequel l'au moins un signal de composante du signal de courant alternatif infrarouge comprend un signal artériel.

11. Appareil selon l'une quelconque des revendications 8 à 10, dans lequel le processeur est également configuré pour :

réaliser un traitement de filtrage sur le signal de lumière verte ; et

déterminer les données de courant alternatif de lumière rouge sur la base du signal de composante du signal de courant alternatif de lumière rouge et d'un signal de lumière verte obtenu après le traitement de filtrage, et déterminer les données de courant alternatif infrarouge sur la base du signal de composante du signal de courant alternatif infrarouge et du signal de lumière verte obtenu après le traitement de filtrage.

12. Appareil selon la revendication 10 ou 11, dans lequel le processeur est également configuré pour :

réaliser une comparaison sur la base de l'au moins un signal de composante du signal de courant alternatif de lumière rouge et du signal de lumière verte, et déterminer que des données correspondant à au moins un signal de composante, du signal de courant alternatif de lumière rouge, le plus proche du signal de lumière verte sont les données de courant alternatif de lumière rouge ; et

réaliser une comparaison sur la base de l'au moins un signal de composante du signal de courant alternatif infrarouge et du signal de lumière verte, et déterminer que des données correspondant à au moins un signal de composante, du signal de courant alternatif infrarouge, le plus proche du signal de lumière verte sont les données de courant alternatif infrarouge.

**13.** Appareil selon l'une quelconque des revendications 8 à 12, dans lequel

les données de courant continu de lumière rouge sont une valeur moyenne, une valeur maximale, une valeur minimale, ou une médiane des au moins deux signaux de courant continu de lumière rouge ; et
les données de courant continu infrarouge sont une valeur moyenne, une valeur maximale, une valeur minimale ou une médiane des au moins deux signaux de courant continu infrarouge.

**14.** Appareil selon l'une quelconque des revendications 8 à 13, dans lequel le processeur est également configuré pour :

déterminer l'oxygène du sang pulsé sur la base des données de courant continu de lumière rouge, des données de courant alternatif de lumière rouge, des données de courant continu infrarouge et des données de courant alternatif infrarouge ; et
interroger une table de comparaison préconfigurée sur la base de l'oxygène du sang pulsé pour déterminer la saturation en oxygène du sang.

FIG. 1a

FIG. 1b

Obtain at least two red light signals, at least two infrared signals, and a green light signal — S201

Determine red light direct current data and a component signal of a red light alternating current signal based on the at least two red light signals; and determine infrared direct current data and a component signal of an infrared alternating current signal based on the at least two infrared signals, where the component signal includes an arterial signal — S202

Determine red light alternating current data based on the component signal of the red light alternating current signal and the green light signal, and determine infrared alternating current data based on the component signal of the infrared alternating current signal and the green light signal — S203

Determine blood oxygen saturation based on the red light direct current data, the red light alternating current data, the infrared direct current data, and the infrared alternating current data — S204

FIG. 2

S201

Determine at least two red light direct current signals and at least two red light alternating current signals based on the at least two red light signals — S301

Determine red light direct current data based on the at least two red light direct current signals — S302

Determine at least two infrared direct current signals and at least two infrared alternating current signals based on at least two infrared signals — S303

Determine the infrared direct current data based on the at least two infrared direct current signals — S304

Determine a component signal of the red light alternating current signal based on the at least two red light alternating current signals; and determine a component signal of the infrared alternating current signal based on the at least two infrared alternating current signals — S305

Determine red light alternating current data based on the component signal of the red light alternating current signal and a green light signal obtained after the filtering processing, and determine infrared alternating current data based on the component signal of the infrared alternating current signal and the green light signal obtained after the filtering processing — S306

Determine pulse blood oxygen based on the red light direct current data, the red light alternating current data, the infrared direct current data, and the infrared alternating current data — S307

Query a pre-configured comparison table based on the pulse blood oxygen to determine blood oxygen saturation — S308

FIG. 3

FIG. 4

FIG. 5

**EP 3 915 476 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103169478 A
- CN 108937957 A
- WO 2016015009 A1
- US 7469157 B2
- US 2016367154 A1